# EUROPEAN PATENT APPLICATION

(11) **EP 0 888 774 A2**
(43) Date of publication of application: **07.01.1999**
(21) Application number: 98202075.2
(22) Date of filing: 23.06.1998
(51) Int. Cl.: A61K 31/355

(54) **Soft gel Coenzyme Q10 formulation**

(30) Priority: 30.06.1997 US 886122
(71) Applicant: Soft Gel Technologies, Inc., Los Angeles, CA 90040 (US)
(72) Inventor: Steele, Don, Westlake, CA 91361 (US)
(74) Representative: Weatherald, Keith Baynes

(57) **Abstract**

A soft gel Coenzyme Q10 formulation and methodology for maintaining Coenzyme Q10 basal blood levels and improving Coenzyme Q10 intestinal tract absorption; the soft gel formulation including Vitamin E and rice bran oil is administered in a 30-100 mg/day dosage, preferably in two equal dosages.

## Description

### BACKGROUND OF THE INVENTION

This invention relates to an improved soft gel formulation of Coenzyme Q10 which increases basal steady state blood levels and the rate of intestinal tract absorption. The soft gel formulation and method of administration has been successfully used in the treatment of heart failure, chronic fatigue syndrome, psoriasis and planter warts.

Coenzyme Q10 (CoQ10 or Ubiquinone) is a large (863.63) gram molecular weight) lipid compound that is produced in the liver and perhaps other body organs. The total body content is estimated to be 1.4 to 1.8 grams depending on the age and the physical fitness of the individual. Although CoQ10 is found in the mitochondria and other organelles of every living cell, it appears to be most abundant in tissues with a high number of mitochondria and a high level of metabolic activity. For example, in the metabolic inactive blood there is approximately 4 mg, in the heart 28 mg, and in the skeletal muscle 1000 mg. The blood acts as a CoQ10 reservoir and transport media between endogenous CoQ10 synthesis in the liver, exogenous CoQ10 absorption from digested food substances in the intestinal tract, and the body cells. Endogenous synthesis appears to be responsible for 56 percent and exogenous sources 44 percent of the body CoQ10 requirement. These numbers are currently being studied and endogenous CoQ10 synthesis may be significantly deficient in the elderly. These deficiencies are not related to the total caloric intake, but rather to the vitamin content of ingested foods. Multiple vitamins are required by the body for the synthesis CoQ10.

CoQ10 requirements of the body are also variable between individuals and are dependent on age, physical activity, and disease. It is estimated that the body CoQ10 utilization is between 5 and 9 mg per day. Intercellular CoQ10 is required for the synthesis of energy and therefore essential for life. Energy synthesis occurs in the mitochondria where CoQ10 provides an electron for the electron transport chain in-the cytochrome system in which adenosine triphosphate (ATP) is synthesized. When CoQ10 gives up an electron for ATP synthesis, it is hydrolysed and then becomes a very potent intercellular antioxidant. When used as an antioxidant, CoQ10 is no longer available to function in the synthesis of ATP. Under conditions of high metabolic stress, environmental sources may become inadequate to meet the body's CoQ10 requirement for ATP synthesis. Under such conditions, dietary CoQ10 supplementation has been shown to be effective. An improved soft gel formulation has uses to treat heart failure, chronic fatigue and patients with psoriasis and planter warts. In all cases, it has been found that the improved soft gel formulation at doses of 30-100 mg/day have been proved to be superior to commercially available 60 mg dry capsules, and existing 100 mg/day soft gel formulations.

An appropriate CoQ10 dosage for a normal individual compared to the dosage necessary for a diseased individual has been difficult to ascertain. Recommended doses of 10 to 30 mg/day were found to be ineffective in patients with significant CoQ10 deficiencies. In the past 15 years, it has become generally accepted that poor intestinal absorption of certain CoQ10 formulations limit its effective use. For this reason we now find 50 and 150 mg tablets or capsules commercially available to the consumer at a considerable higher cost.

Folkers et al (U.S. Patent 4,824,669) addresses a soft gel capsule with CoQ10 and at least one vegetable oil. This formulation was determined to increase blood CoQ10 levels to 2.5 ug/ml compared to 1.6 ug/ml for an equivalent 100 mg dose of dry CoQ10. Many different CoQ10 formulations have appeared which are claimed to increase intestinal absorption. However, intestinal absorption data, collected under near basal conditions, which compare CoQ10 alone in oil with dry CoQ10, are inconclusive.

It is therefore an object of the present invention to provide an improved soft gel formulation of CoQ10 and a methodology of administration that produces a significantly greater basal blood CoQ10 level than existing soft or dry formulations.

It is a further objective of the present invention to provide a soft gel formulation of CoQ10 and methodology of administration that produces greater absorption into the intestines.

### SUMMARY OF THE INVENTION

The present invention comprises a stable and nontoxic soft gel coenzyme Q10 formulation and methodology of administration for maintenance of high coenzyme Q10 basal blood levels and increased coenzyme Q10 levels in the intestinal tract. A preferred soft gel formulation includes coenzyme Q10, Vitamin E and rice bran oil. The preferred soft gel Coenzyme Q10 formulation is administered twice a day in dosages of about 30 mg.

### DETAILED DESCRIPTION OF THE PREFERRED EMBODIMENTS

Coenzyme Q10 is provided in a dosage of approximately 30-100 mg per day in a soft gel formulation including rice bran oil and Vitamin E. A preferred formulation and methodology entails the administration of about 60 mg of the formulation dispensed twice a day (30 mg/dose). The first administration may occur in the morning between 6:30 AM and 7:30 AM, and a similar dosage administration occurs between 2:00 PM and 3:00 PM in the afternoon approximately six to eight hours after the first dosage. Dosages of 30-100 mg/day of the improved soft gel formulation dosages during the day have shown superior results.

The unique formulation (formulation A) was administered during a study to Group I (30 mg CoQ10, 30 IU Vitamin E, and rice bran oil). A second group was administered a dry formulation B (30 mg dry CoQ10, 30 lU Vitamin E, and rice flour), and a third Group III was administered a placebo, formulation C.

During a five day control period, two (2) blood samples were taken on day one and five from 36 screened subjects.

Each volunteer took two doses of their respective formulations daily (7:00 AM and 7:00 PM) for the next thirty (30) days. Venous blood samples were taken on days 15 and 30 of the active phase of this study. An additional blood sample was taken on day 31, five (5) hours after taking a single dose of their respective formulation, and fifteen (15) days after the volunteers quit taking their respective formulations. All blood samples were taken while the volunteers were in a near basal state, i.e., between 7:00 and 7:30 AM, after sleeping at least eight (8) hours, and before any food or CoQ10 ingestion. CoQ10 levels were measured in whole blood. CoQ10 analysis was done using an HPLC method.

Within and between Groups statistical analysis was determined using the paired t-test for group mean differences for equal N values. All group data are presented as means and standard deviations. P-values between groups or conditions equal to or less than 0.01 were accepted as significantly different.

Individual volunteer and Group blood CoQ10 levels are presented in Tables I, II and III. Group I, II and III blood CoQ10 levels and statistical differences within and between groups are presented in Table IV.

Although a considerable variation between individual blood CoQ10 levels were seen in each study group (Tables I, Il, and III), the Group mean control values were not significantly different between groups (GI = 0.85+-0.16, GII = 0.85+-0.16, and GIII = 0.83+-0.21 ug/ml: Table IV).

After 15 days of CoQ10 or placebo consumption, considerable basal blood CoQ10 variations within and between groups were present (Tables I, II and III). Group I and II mean values (GI = 2.13+-0.29; GII = 1.42+-0.22) were increased significantly (p<0.001) compared to the initial control levels or that of the placebo Group (GIII = 0.93+-0.21). Group I mean levels were significantly (p<0.001) greater than that of Group II (Table IV).

At the 30 day intervals, Group I and II mean blood CoQ10 levels only increased 10 to 12 percent above those of the 15 day interval (Tables I, II, and IV). The within group difference between the 15th and 30th day was not significant (p>0.6), however the mean differences between Groups I (2.26+-0.21) and II (1.53+-0.21) remained significantly greater in Group I, (p<0.001). Also, the Group mean differences between Groups I and II compared to Group III remained significantly elevated (p<0.001). Differences between the 15th and 30th day levels in Group I remains fairly constant after 15 days.

To determine the intestinal CoQ10 absorption over a five hour interval, venous blood samples were taken between 7:00 and 7:30 AM after taking one dose (30 mg CoQ10) between 2:00 and 2:30 AM. After five hours, blood CoQ10 was increased in Group I and II, but not Group m (Table IV). The Group I mean level (2.74+-0.21) was significantly (p<0.001) greater than of Group II (1.72+-024). The Group III mean value (0.84+-0.13) was not significantly different compared to any of the previous Group III levels. To gain an estimate of the CoQ10 absorption over the five hour interval, the total blood CoQ10 content was calculated from the ug/ml and the total blood volume product (ug/ml × Blood Volume). Total blood volume was estimated from an age, height, weight, and sex nomogram. The CoQ10 absorption was calculated in ug/ml/minute (Table lV). The between volunteer absorption rate was variable (Group I = 20.5% and Group II = 26.9%) in both Groups (Table V). Group I mean absorption rate (9.3+-1.9 ug/ml/min) was significantly (p<0.001) greater than that of Group II (3.4+-0.92 ug/ml/min). These data indicate that the 30 mg CoQ10 in rice bran oil and Vitamin E has a 260 percent greater peak absorption than that of the dry formulation.

Individual and Group mean basal blood CoQ10 levels in Group I and II returned to or near the initial control levels (Tables I-III and IV) after 15 days of no supplementation. Differences between Groups were not significant between Groups nor between the post 15 day and control intervals.

The bioavailability or intestinal absorption of CoQ10 has been a major controversy in the international CoQ10 research community. Previous data indicate that only 1 to 3 percent of a dry CoQ10 formulation is absorbed through the lacteals in the intestines and appear in the blood over a twelve hour interval. In generally, blood levels of 1.2 to 1.6 ug/ml have been reported when taking 30 to 60 mg/day dry CoQ10 formulation for 30 days. It has been reported that when a dry CoQ10 formulation is taken with a fat such as peanut butter, steady state blood levels of 2.0 to 2.8 ug/ml are measurable. Multiple clinical trials were conducted in the United States and Europe using the Folkers (U.S. Patent No. 4,824,669) soft gel. With a dosage of 100 mg/day multiple investigators have reported group mean blood levels of 2.3 to 3.5 ug/ml depending on the laboratory conducting the measurement.

The steady state basal blood CoQ10 level of 2.1 to 2.26 ug/ml found in Group I with only a 60 mg daily CoQ10 supplementation is within the lower range of that reported for the Folkers soft gel at a dose of 100 mg/day. The 1.4 to 1.5 ug/ml found in Group II (60 mg dry CoQ10 per day) is significantly (p<0.001) less than the 2.26 ug/ml found in Group I with 60 mg/day dosage (Table IV). However, it is similar to that previously reported for dry CoQ10 formulations. In this study, 10 of 12 volunteers in group I indicated improved energy levels 3 to 5 hours after taking the soft gel CoQ10 formulation. All volunteers in this group expressed a significant lack of energy in the early AM, and reduced energy with no energy surge after stopping CoQ10 supplementation. Only 4 of 12 volunteers in Group II felt a significant energy improvement when taking the dry CoQ10 formulation. However, 8 of 12 felt a lack of energy within five days after stopping CoQ10 supplementation. These observations indicate that the higher blood levels attained in Group I were positively (+83%) related to the well being of these normal volunteers compared to only 33% of the Group II normal volunteers.

The 9.3+-1.9 µg/ml/min peak absorption rate at five hours post 30 mg CoQ10 ingestion found in Group I compared to the 3.42 µg/ml/min found in Group II is significant. These data show that over a five hours' interval, approximately 9 percent (2.7 mg) of the 30 mg CoQ10 soft gel formulation was absorbed compared to 3 percent (1.0 mg) of the 30 mg dry CoQ10 formulation (Table V). The peak absorption rate of the Folkers soft gel five hours after taking 30 mg in approximately 5.1 µg/min, 2.1 mg total absorption, 7 percent of the 30 mg dose.

The importance of the results found for the inventive soft gel is that one 30 mg capsule will provide approximately 50 percent, and two capsules, 100 percent of the daily CoQ10 requirements of a normal sedentary individual. It would take at least three of the dry 30 mg CoQ10 capsules to produce the same affects as one soft gel, and six to produce the same affect as two 30 mg soft gel capsules. Regardless of the absorption mechanism, the significantly higher basal blood CoQ10 levels (167%) and the 273 percent greater absorption rate found in Group I show that the soft gel formulation is indeed a superior product to the dry CoQ10 formulation. This may be especially true for those individuals whose daily CoQ10 requirement is elevated due to high physical activity, an increased use of CoQ10 as an antioxidant, and diseases associated with known CoQ10 deficiencies.

Cellular CoQ10 content is a function of the number and quality of the cellular mitochondria. For example, the failing heart muscle has 2.2 µg CoQ10 per mg tissue and a blood CoQ10 deficiency (0.3-0.5 µg/ml). The normal hearts have 4.2 µg/mg tissue, and a normal blood level (0.8-1.2 µg/ml). The conditioned heart has 6.3 µg/gm tissue, and a low basal blood level (0.5-0.6 µg/ml). These are evidence that supplemental CoQ10 enters the cell. This observation has also been reported for skeletal muscle of trained and non-trained athletes. The fact that 83 percent of Group I volunteers experienced more energy with the higher blood CoQ10 levels than did Group II (33%) with lower blood levels, and that almost all of volunteers (90%) complained of reduced energy when CoQ10 supplementation was stopped suggest that a higher blood CoQ10 favors better CoQ10 transport into the cell. If the exogenous CoQ10 was not going into the cell, the volunteers would not experience such positive energy changes.

The subjective and objective responses to supplemental CoQ10 in the normal individual appear more rapidly compared to that of the physically unfit or the diseased individual with a CoQ10 deficiency. The most probable reason for this observation is that the metabolic machinery (mitochondria) is viable in the non-diseased normal volunteer, whereas the mitochondria are atrophied in the cells of deconditioned and diseased individuals. Therefore, it takes time in the diseased individual to build up the mitochondria to a more normal activity level and to normalize their distribution in the organ system involved.

In summary, it is statistically proven that the soft gel CoQ10 formulation used (60 mg/day) is superior to the dry CoQ10 formulation, and prior art soft gel formulations, relative to (1) increasing the basal steady state blood level and (2) to increasing the rate of intestinal absorption of CoQ10.

Changes may be made in construction, operation and arrangement of various elements, steps and procedures described herein without departing from concept and scope of the invention as defined in the following claims.

**TABLE I**

| **Group I** **Basal and Dynamic Blood CoQ10 Levels** **(Soft Gel)** | | | | | |
|---|---|---|---|---|---|
| **Subjects** | **Control** | **15 Days** | **30 Days** | **5 Hour Post 30 mg** | **15 Day Post CoQ10** |
| 1 | 0.900 | 2.000 | 2.100 | 2.600 | 0.900 |
| 2 | 0.900 | 1.900 | 2.200 | 2.700 | 0.800 |
| 3 | 0.800 | 1.800 | 2.300 | 2.800 | 0.800 |
| 4 | 0.700 | 1.700 | 1.900 | 2.500 | 1.000 |
| 5 | 0.900 | 1.900 | 2.100 | 2.700 | 1.000 |
| 6 | 0.700 | 2.600 | 2.600 | 3.100 | 0.800 |
| 7 | 0.900 | 2.500 | 2.400 | 2.800 | 0.900 |
| 8 | 1.300 | 2.100 | 2.200 | 2.600 | 1.400 |
| 9 | 0.800 | 2.200 | 2.400 | 3.100 | 0.800 |
| 10 | 0.800 | 1.700 | 2.100 | 2.600 | 0.700 |
| 11 | 0.700 | 1.800 | 1.900 | 2.400 | 0.700 |
| 12 | 0.800 | 1.800 | 2.000 | 2.700 | 0.900 |
| | | | | | |
| Mean | 0.850 | 2.130* | 2.260* | 2.740* | 0.850 |
| S.D. | 0.160 | 0.290 | 0.210 | 0.210 | 0.180 |

| | | | | | |
|---|---|---|---|---|---|
| ug/ml: *p<0.001 | | | | | |

**TABLE II**

| **Group II** **Basal and Dynamic Blood CoQ10 Levels** **(Dry CoQ10)** | | | | | |
|---|---|---|---|---|---|
| **Subjects** | **Control** | **15 Days** | **30 Days** | **5 Hour Post 30 mg** | **15 Day Post CoQ10** |
| 1 | 1.000 | 1.600 | 1.700 | 1.900 | 1.200 |
| 2 | 0.700 | 1.200 | 1.500 | 1.700 | 0.800 |
| 3 | 0.800 | 1.200 | 1.400 | 1.600 | 1.000 |
| 4 | 0.700 | 1.400 | 1.200 | 1.400 | 0.700 |
| 5 | 1.200 | 1.700 | 1.800 | 2.100 | 1.300 |
| 6 | 0.800 | 1.400 | 1.400 | 1.700 | 0.700 |
| 7 | 0.600 | 0.900 | 1.200 | 1.300 | 0.700 |
| 8 | 0.800 | 1.400 | 1.400 | 1.500 | 1.800 |
| 9 | 0.800 | 1.500 | 1.600 | 1.800 | 0.900 |
| 10 | 0.900 | 1.700 | 1.800 | 2.000 | 0.800 |
| 11 | 0.900 | 1.600 | 1.700 | 2.000 | 0.100 |
| 12 | 1.000 | 1.400 | 1.300 | 1.600 | 0.900 |
| | | | | | |
| Mean | 0.850 | 1.420* | 1.500* | 1.720* | 0.910 |
| S.D. | 0.160 | 0.220 | 0.210 | 0.240 | 0.220 |

| | | | | | |
|---|---|---|---|---|---|
| ug/ml: *p<0.001 | | | | | |

**TABLE III**

| **Group III** **Basal and Dynamic Blood CoQ10 Levels** **(Placebo)** | | | | | |
|---|---|---|---|---|---|
| **Subjects** | **Control** | **15 Days** | **30 Days** | **Hour Post 30 mg** | **15 Day Post CoQ1O** |
| 1 | 0.800 | 0.900 | 2.100 | 2.600 | 0.900 |
| 2 | 1.200 | 1.400 | 2.200 | 2.700 | 0.800 |
| 3 | 0.900 | 1.000 | 2.300 | 2.800 | 0.800 |
| 4 | 0.600 | 0.700 | 1.900 | 2.500 | 1.000 |
| 5 | 0.700 | 0.800 | 2.100 | 2.700 | 1.000 |
| 6 | 0.900 | 1.100 | 2.600 | 3.100 | 0.800 |
| 7 | 0.700 | 0.800 | 2.400 | 2.800 | 0.900 |
| 8 | 0.800 | 1.000 | 2.200 | 2.600 | 1.400 |
| 9 | 1.300 | 1.100 | 2.400 | 3.100 | 0.800 |
| 10 | 0.600 | 0.800 | 2.100 | 2.600 | 0.700 |
| 11 | 0.700 | 0.800 | 1.900 | 2.400 | 0.700 |
| 12 | 0.800 | 1.200 | 2.000 | 2.700 | 0.900 |
| | | | | | |
| Mean | 0.830 | 0.930+ | 0.960+ | 0.840+ | 0.920+ |
| S.D. | 0.210 | 0.210 | 0.140 | 0.130 | 0.180 |

| | | | | | |
|---|---|---|---|---|---|
| ug/ml: +p>0.6 (ns) | | | | | |

**TABLE IV**

| **Summary of Group Basal and Dynamic Blood CoQ10 Levels** **(Within and Between Group Analysis)** | | | | | |
|---|---|---|---|---|---|
| **Groups** | **Control** | **15 Days** | **30 Days** | **5 Hour Post 30 mg** | **15 Day Post CoQ10** |
| I | 0.850+ | 2.130* | 2.260* | 2.740* | 0.850+ |
| Soft Gel | 0.160 | 0.290 | 0.210 | 0.210 | 0.180 |
| II | 0.850+ | 1.420* | 1.530* | 1.720* | 0.910+ |
| Dry | 0.160 | 0.220 | 0.210 | 0.249 | 0.220 |
| III | 0.830+ | 0.930* | 0.960* | 0.840* | 0.920+ |
| Placebo | 0.210 | 0.210 | 0.140 | 0.130 | 0.180 |
| Means/S.D.: ug/ml | | | | | |

| | | | | | |
|---|---|---|---|---|---|
| * p<0.001 (I-vs-II & III; II-vs-III) | | | | | |
| + p>060 (I-vs-II & III; II-vs-III) | | | | | |

**TABLE V**

| **Peak CoQ10 Absorption Rate** **(Five Hours Post 30 mg CoQ10** **(ug/ml/min)** | | |
|---|---|---|
| **Subjects** | **Group I** | **Group II** |
| 1 | 8.300 | 3.300 |
| 2 | 7.600 | 3.300 |
| 3 | 8.800 | 3.800 |
| 4 | 10.900 | 3.100 |
| 5 | 9.900 | 5.200 |
| 6 | 8.600 | 4.600 |
| 7 | 7.400 | 1.700 |
| 8 | 6.700 | 1.900 |
| 9 | 11.700 | 4.300 |
| 10 | 9.800 | 3.500 |
| 11 | 8.600 | 3.300 |
| 12 | 13.800 | 4.100 |
| | | |
| Mean | 9.340* | 3.400 |
| S.D. | 1.920 | 0.920 |

| | | |
|---|---|---|
| * p>0.001 for I-vs-II | | |

## Claims

1. A method for improved absorption of Coenzyme Q10 into an intestinal tract and maintenance of Coenzyme Q10 basal blood levels comprising administration of approximately 30-100 mg/day of a soft gel formulation of Coenzyme Q10 and Vitamin E.

2. A method for improved absorption of Coenzyme Q10 into an intestinal tract and maintenance of Coenzyme Q10 basal blood levels as claimed in claim 1, said soft gel formulation further comprising rice bran oil.

3. A method for improved absorption of Coenzyme Q10 into an intestinal tract and maintenance of Coenzyme Q10 basal blood levels as claimed in claim 1, wherein said soft gel formulation is administered in two daily dosages.

4. A method for improved absorption of Coenzyme Q10 into an intestinal tract and maintenance of Coenzyme Q10 basal blood levels as claimed in claim 3, wherein said two daily dosages comprise approximately 30 mg Coenzyme Q10 per dosage.

5. A method for improved absorption of Coenzyme Q10 into an intestinal tract and maintenance of Coenzyme Q10 basal blood levels as claimed in claim 3, wherein said two daily dosages are administered approximately six to eight hours apart.

6. A method for improved absorption of Coenzyme Q10 into an intestinal tract and maintenance of Coenzyme Q10 basal blood levels as claimed in claim 1, wherein said formulation is administered orally.

7. A method for improved absorption of Coenzyme Q10 into an intestinal tract and maintenance of Coenzyme Q10 basal blood levels as claimed in claim 2, wherein said formulation is administered in approximately two equal dosages.

8. A soft gel formulation for absorption of Coenzyme Q10 into an intestinal tract and maintenance of Coenzyme Q10 basal blood levels comprising Coenzyme Q10 and Vitamin E.

9. A soft gel formulation for absorption of Coenzyme Q10 into an intestinal tract and maintenance of Coenzyme Q10 basal blood levels as claimed in claim 8, further comprising rice bran oil.

10. A soft gel formulation for absorption of Coenzyme Q10 into an intestinal tract and maintenance of Coenzyme Q10 basal blood levels as claimed in claim 8, wherein said formulation comprises 30-100 mg Coenzyme Q10 and 30-100 IU Vitamin E.

11. A soft gel formulation for absorption of Coenzyme Q10 into an intestinal tract and maintenance of Coenzyme Q10 basal blood levels as claimed in claim 10, wherein said formulation is administered in approximately equal dosages twice a day.
